# EUROPEAN PATENT SPECIFICATION

(11) **EP 3 737 318 B1**
(45) Date of publication and mention of the grant of the patent: **21.10.2026**
(21) Application number: 19705599.9
(22) Date of filing: 11.01.2019
(51) Int. Cl.: A61B 34/00, A61B 34/20, A61B 34/30

(54) **ROBOTIC SURGICAL CONTROL SYSTEM**
CHIRURGISCHES ROBOTERSTEUERUNGSSYSTEM
SYSTÈME DE COMMANDE CHIRURGICAL ROBOTISÉ

(30) Priority: 12.01.2018 US 201862616700 P
(43) Date of publication of application: 18.11.2020
(73) Proprietor: Globus Medical, Inc., Audubon, PA 19403 (US)
(72) Inventor: LARK, James, D., Bloomfield, MI 48324 (US); SCALES, John, S., Ann Arbor, MI 48105 (US); BONO, Peter, L., Bingham Farms, MI 48025 (US); LORD, Thomas J., South Milwaukee, WI 53172 (US)
(74) Representative: Morabito, Sara
(86) International application number: PCT/US2019/013225
(87) International publication number: WO 2019/140214

(56) References cited:
- WO-A1-2013/192598
- US-A1- 2015 100 066
- US-A1- 2017 245 940
- US-A1- 2017 296 292

## Description

### FIELD OF THE INVENTION

The invention relates to robotic surgical procedures and, more specifically, to a software system configuring a computer system that controls the movements of a multi-axial robot to perform an orthopedic surgical procedure.

### BACKGROUND INFORMATION

The performance of an orthopedic surgical procedure on the spine with the assistance of a robot is known in the art. However, the surgeries that have been previously performed are merely small portions of the surgery, or have been small steps in the overall completion of the surgery. Thus, the known control systems configured by software for controlling the movements of the robot are deficient for complex and delicate procedures such as bone removal, disc removal and the like.

US2015/100066 and WO2013/192598 disclose surgical systems.

Thus, the present disclosure provides a software configured control system which overcomes the disadvantages of prior art robot surgical systems. The robotic surgical control system of the present disclosure not only provides for relative ease in the setup of the procedure and control over the robot during the actual operation, it also provides warnings and monitoring of the procedure to detect, warn and prevent injury to a patient undergoing the procedure.

### SUMMARY OF THE INVENTION

The invention is defined by the appended independent claim. Further embodiments of the invention are illustrated in the dependent claims. The methods described herein do not form part of the invention, but are useful for understanding the invention.

### SUMMARY OF THE DISCLOSURE

Briefly, the disclosure involves a system and method for controlling the movements of a multi-axis robot to perform a surgery at least on the spinal area of a human *in vivo.* The system includes controls and software coding to cause the robot to move in desired patterns to complete a surgery, which may include bone, disc and tissue removal, and may also include insertion of hardware for fusing adjacent bony structures.

Accordingly, it is an objective of the present disclosure to provide a system for controlling a multi-axis robot for performance of a surgical procedure.

It is a further objective of the present disclosure to provide software for controlling the movements of a multi-axis robot for performance of a surgical procedure.

It is yet a further objective of the present disclosure to provide a method for controlling a multi-axis robot for the purpose of performing a surgical procedure.

Other objectives and advantages of this disclosure will become apparent from the following description taken in conjunction with the accompanying drawings wherein are set forth, by way of illustration and example, certain embodiments of this disclosure. The drawings constitute a part of this specification, include exemplary embodiments of the present disclosure, and illustrate various objects and features thereof.

### BRIEF DESCRIPTION OF THE FIGURES

Figure 1 is a screen shot illustrating the opening screen of the present system;
Figure 2 is a screen shot illustrating a login screen for the present system;
Figure 3 is a screen shot illustrating a patient selection screen for the present system;
Figure 4 is a screen shot illustrating a patient information screen for the present system;
Figure 5 is a screen shot illustrating a procedure selection screen for the present system;
Figure 6 is a screen shot illustrating a cut planning screen for the present system;
Figure 7 is a screen shot illustrating a cutting tool path screen for the present system;
Figure 8 is a screen shot illustrating a screw insertion planning screen for the present system;
Figure 9 is a screen shot illustrating another screw insertion planning screen for the present system;
Figure 10 is a screen shot illustrating a (DICOM) digital imaging and communications in medicine screen for the present system for viewing the patient's anatomy;
Figure 11 is a screen shot illustrating a DICOM slice view screen for the present system;
Figure 12 is a screen shot illustrating an operation preview screen for the present system;
Figure 13 is a screen shot illustrating an operation preview screen in slice format for the present system;
Figure 14 is a general flow diagram for the present system; and
Figure 15 is a view of the robotic control system including one embodiment of the robot.

Other objectives and advantages of this disclosure will become apparent from the following description taken in conjunction with the accompanying drawings wherein are set forth, by way of illustration and example, certain embodiments of this disclosure. The drawings constitute a part of this specification and include exemplary embodiments of the present disclosure and illustrate various objects and features thereof.

### DETAILED DESCRIPTION OF PREFERRED EMBODIMENTS

While the present disclosure is susceptible of embodiment in various forms, there is shown in the drawings and will hereinafter be described a presently preferred embodiment with the understanding that the present disclosure is to be considered an exemplification of the disclosure and is not intended to limit the disclosure to the specific embodiments illustrated.

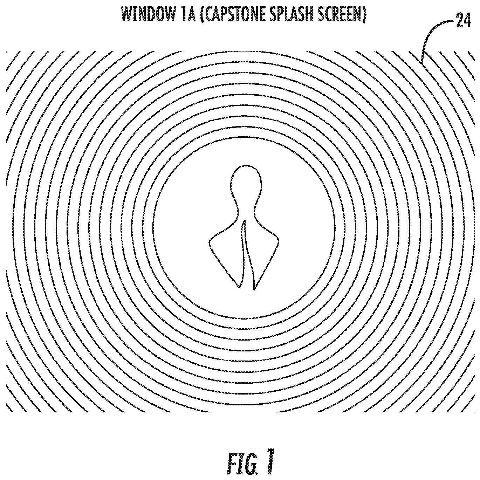

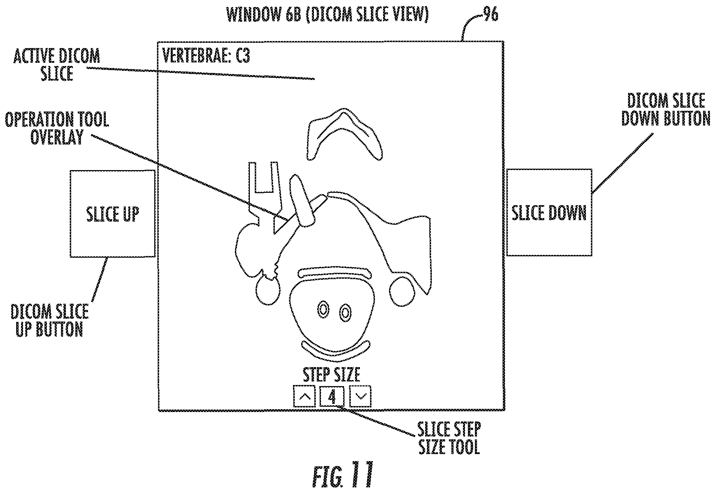

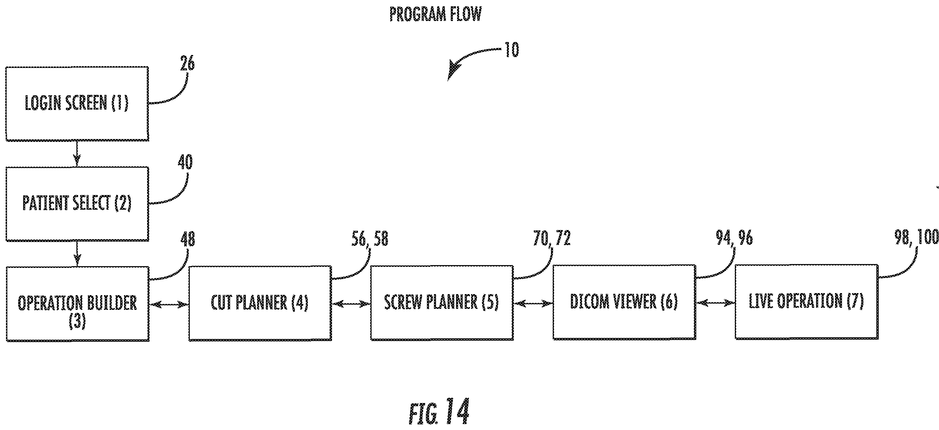

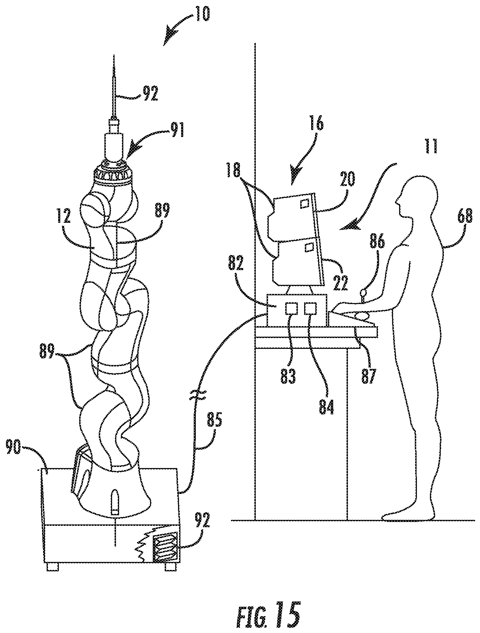

Referring generally to Figs. 1-15, a robotic surgical system 10 that includes a computer system 11 operably connected or interfaced with a multi-axis robot 12 for controlling the movements of components of the multi-axis robot 12 to perform a surgical procedure on a human patient 14 *in vivo* is illustrated. The computer system 11 includes a user interface 16, shown as two touchscreen displays 18. The primary visual display 20 is positioned above the work display 22, and may be provided with a horizontal tilt toward the operator 68 to facilitate easier tactile interaction. It is contemplated that these touchscreen displays have built-in multi touch support for scrolling, zooming and rotating viewpoints and target objects, in addition to standard click based selection functionality. The displays 20, 22 are operable to display one or more digital images from one or more image files in storage 83. The computer system 11 includes a computer 82 having storage 83 and a processor 84. The computer 82 is operably connected to the robot 12 wirelessly or by suitable wiring 85. The storage 83 can have a primary storage (commonly referred to as memory) and/or a secondary storage device that can be read by the processor 84 to transfer the digital images between the primary storage to the secondary storage for subsequent long-term storage as an image file, such as a jpeg file. Primary and secondary storage are herein referred to as storage collectively, and the term storage can include one or both primary and secondary storage. It is to be understood that the image file can be transferred wirelessly between primary storage and long-term storage. It is also to be understood that long term storage can be remote, for example, cloud storage or other remote storage.

While operational information can be input by a user such as a surgeon via the display 20, 22, it can also be input by other input devices such as a keyboard 87, joystick or mouse 86 and a touchscreen display 18. The information input can be by a user touching the screen on a display 20, 22 with a finger, stylus or other suitable implement. Based upon the commands input into the computer 82, the computer outputs electrical signals to the multi-axis robot 12 to cause the motors in the robot to move the robot's 12 component arms 89 to a desired position at a desired speed. A tool holder 91 is also associated with the robot 12 for holding a suitable surgical tool 92. The motors (not shown) of the robot may be servos or any other type of motor suitable to provide feedback to the computer system 11 and thus the control system 10 to ensure that the movements of the various robotic arms 89 are moving as commanded by the control system. Thus, it is desirable that the robotic motors are provided with, but not limited to, encoders to provide the feedback signals to the control system 10. In this manner, the control system 10 can modify the electrical signals to the robot 12 to provide the desired level of control needed for surgical procedures. In addition, the robot 12 may be provided with torque sensors, G-force sensors, speed sensors, orientation sensors and the like to provide additional feedback data to the control system 10 to further enhance control of the robot's movements.

In at least one embodiment, the multi-axis robot 12 is provided with a mobile base assembly 90. The mobile base assembly 90 allows the control system 10 to reposition the robot 12 for a more desirable position for completing the desired operation based upon fiducial landmarks in the patient's anatomy, which may be marked to provide electrical, magnetic induced or other types of signals that allow the control system to position the robot. These movements may be based upon limitations in the robot's ability to move in a desired trajectory or the like. In at least one embodiment, the base assembly 90 is provided with Mecanum wheels 92, which allow movement of the robot in any direction without rotation of the wheel about a vertical axis. Like the robot axes, the Mecanum wheel should be provided with motors and/or sensors that provide feedback to the control system 10, which has the capability to monitor the movement, and alter the movement, in real time based upon discrepancies such as wheel slippage and the like.

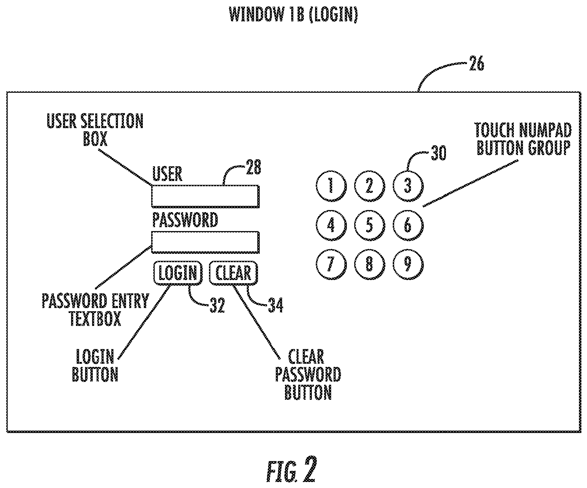

Referring to Figures 1 and 2, the startup and login screens are illustrated. The startup screen 24 is initially viewed when the system is started. Once started, the login screen 26 becomes viewable. To login, the user or operator 68 selects their name from a list of registered users and places their name in the user box. Thereafter, the operator 68 enters a numeric or other suitable password to open the system using the keypad 30 or keyboard 87. The data is entered into the computer using the login button 32, or cleared with the clear button 34. It should also be noted that other types of logins may be utilized without departing from the scope of the disclosure, which may include, but should not be limited to, retina scans, facial scans, fingerprint scans, RFID, and other suitable methods of determining the true identity of an operator 68.

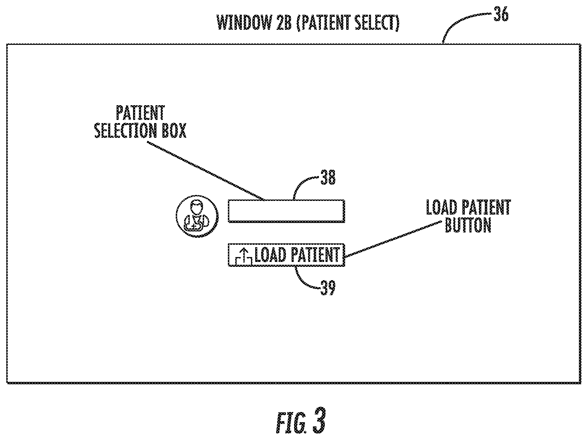

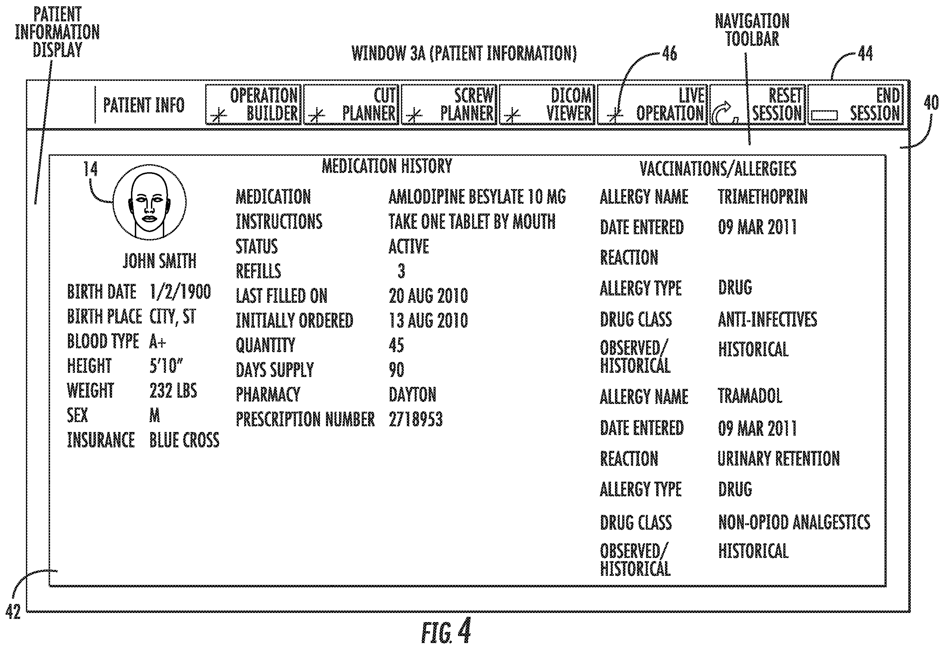

Referring to Figure 3, the patient selection screen is illustrated. To select a patient, the operator 68 may type in or scroll through a list of preloaded patient profiles. The patient's name is then entered into the patient selection box 38 and the load patient button 39 selects the named patient. Once selected, the patient's imaging and medical data (Figure 4) are available for the operator 68, and the system progresses to the next screen.

Referring to Figure 4, the patient information screen 40 is illustrated. In this screen, patient details 42 are viewed on the work display 22. Various surgical procedure types 46 are listed in the navigation toolbar 44 for selection by the operator 68.

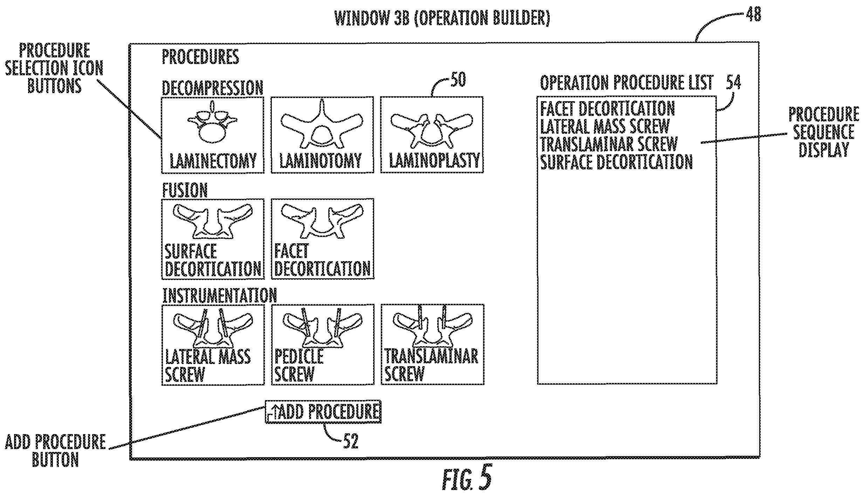

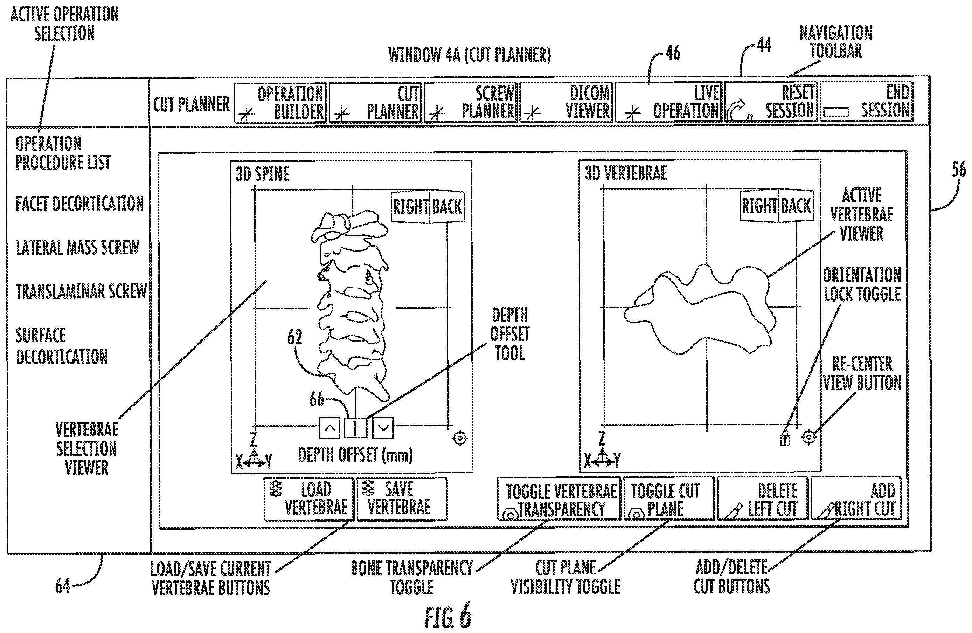

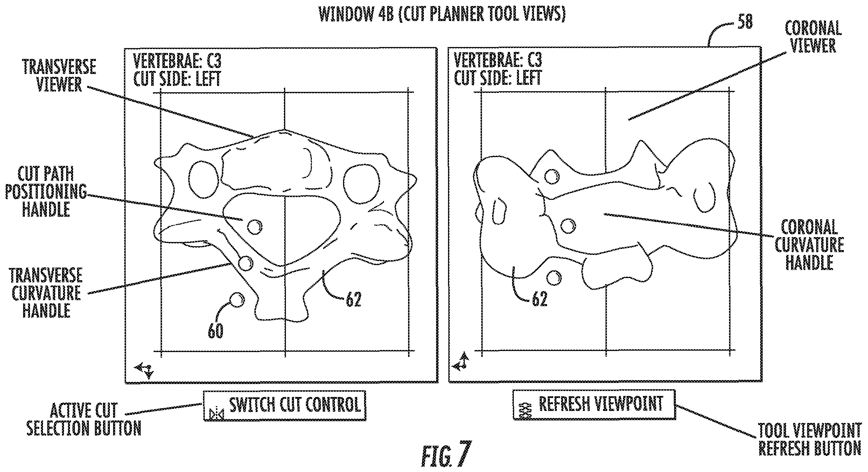

Referring to Figure 5, the operation builder screen 48 is illustrated. Each surgical procedure 46 is provided with picture icons 50. Selection of the desired procedure and pressing the add procedure button 52 appends the sequence of operations in the Operation Procedure List 54 with the steps associated to the selected procedure. The selections made on this screen determine available options on planner screens, see Figures 6 and 7. Pressing procedure types 46 on the navigation toolbar 44 (Figure 4) allows the operator 68 to toggle to and from the operation builder screen 48 throughout operation of the system 10.

Referring to Figures 6 and 7, the cut planner screens 56, 58 are illustrated. In these screens 56, 58, the system 10 allows the operator 68 to select vertebrae 62 and edit cutter paths 60 for the procedure. A proposed surgical tool path 60 is input by the operator as described above on a screen on at least one of the displays 20, 22. The specific procedure to be edited is selected from the Operation Procedure List 64 on the left side of the screen. Each procedure can include operations on any number of vertebrae 62. Tool selection buttons 66 allow the user to select the type of tool to be used for cutting. Each tool selected has an associated tool offset length and diameter to automatically offset and establish the cutting depth of the tool relative to the tool path chosen and directed by the operator 68 for the procedure. All tools chosen are stored for tool path generation. The navigation bar 44 allows the operator 68 to toggle into and out of this screen throughout the procedure as desired. In this manner, the operator 68 can change the surgical tool paths and tools used for the procedure as desired. The on screen view of vertebrae 62 can be rotated and enlarged to allow the surgical tool path 60 to be placed as desired by the operator 68. In this manner, the operator 68 can view the surgical tool path 60 from various angles, and can zoom in on specific features that are desired to remain or be cut away.

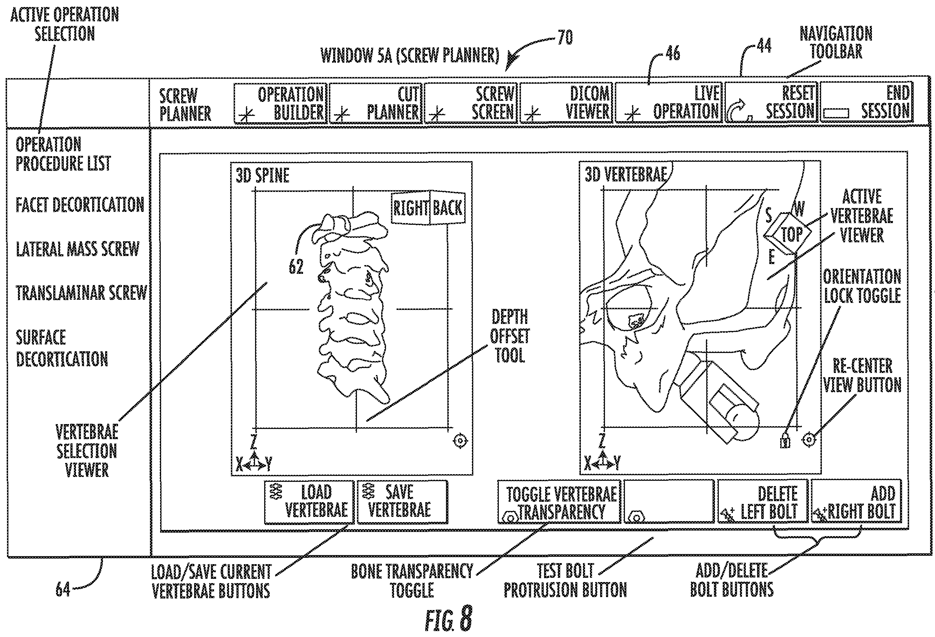

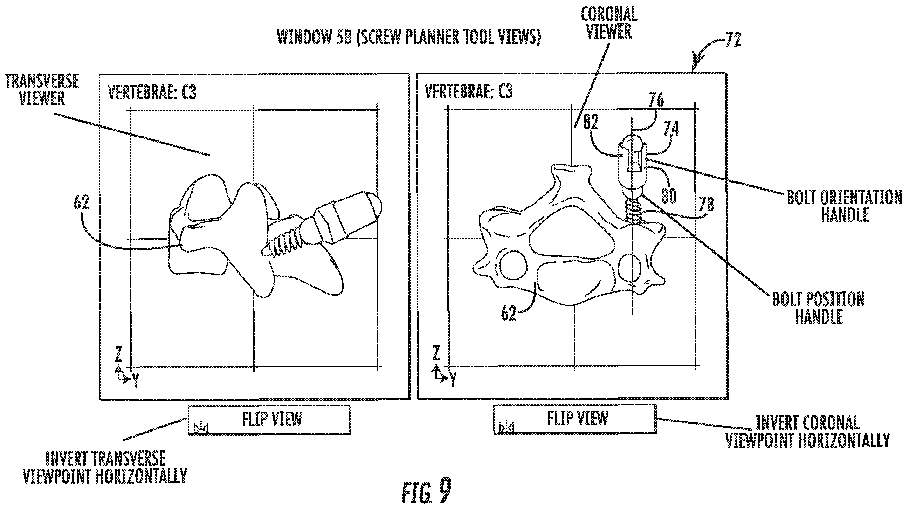

Referring to Figures 8 and 9, planning the insertion of screw screens 70, 72 are illustrated. In these screens, the system 10 allows the operator 68 to edit a specific procedure as selected from the Operation Procedure List 54 (Figure 5) on the right side of the screen. This screen allows the operator 68 to select a desired vertebrae 62 for examination, rotation and positioning, to allow an axis of insertion 76 to be oriented in the vertebrae. Each procedure can include operations on any number of vertebrae, and may include the insertion of pedicle screws 74. Pedicle screws 74 typically include a threaded shank 78 and tulip portion 80. The tulip portion 80 is typically formed to have two upright arms 82 for accepting a rod member (not shown) for connecting multiple screws together to allow fusion of the vertebrae 62 to adjacently positioned vertebrae 62. The pedicle screw 74 is connected to a tube or non-tubular member (not-shown) that immobilizes and orients the upright arms 82 of the tulip portion 80, while engaging the shank 78 so that the screw can be inserted along an axis 76 chosen by the operator 68, and to a desired depth in the vertebrae 62 with the upright arms 82 oriented to allow a rod to be inserted across multiple pedicle screws 74. The tool 92 for inserting the screw, like the other tools 92 (often referred to as an effector), includes a length and a diameter (that may change over its length) stored in the tool offsets, whereby the system knows the length and diameter of the tool for planning and insertion of the screw. This construction thereby provides warnings and notices to the operator 68 if there is interference with the chosen path, or if the screw will protrude from the bone in an undesirable manner. In one embodiment, the control system 10 utilizes the volume of the vertebrae to predict break out or collision to protect the patient from injury, and may also track nerve space, other vertebrae, other tools and sensitive tissue areas of the patient to avoid injury. It should also be noted that, while pedicle screws are illustrated herein, any type of bone screw may be inserted in a similar manner without departing from the scope of the disclosure.

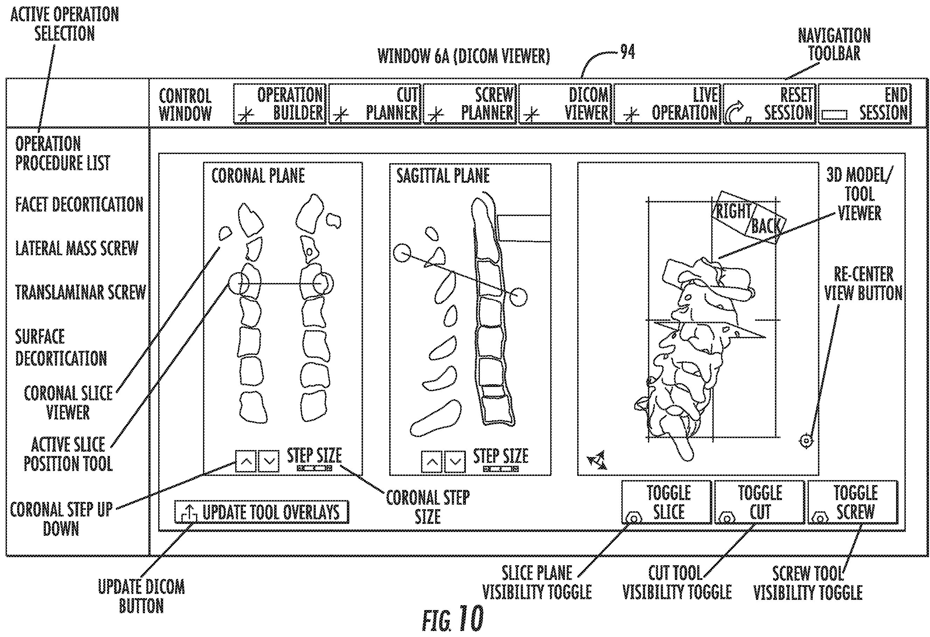

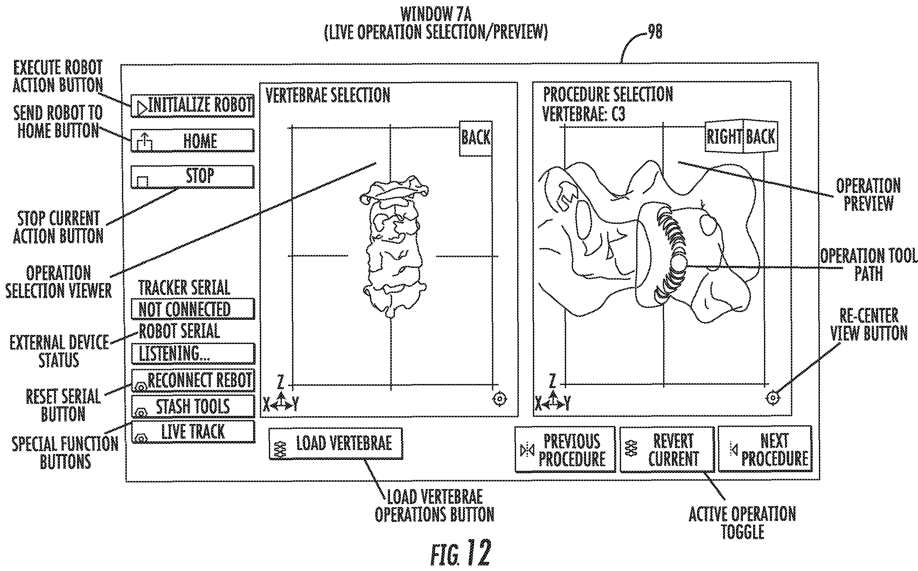

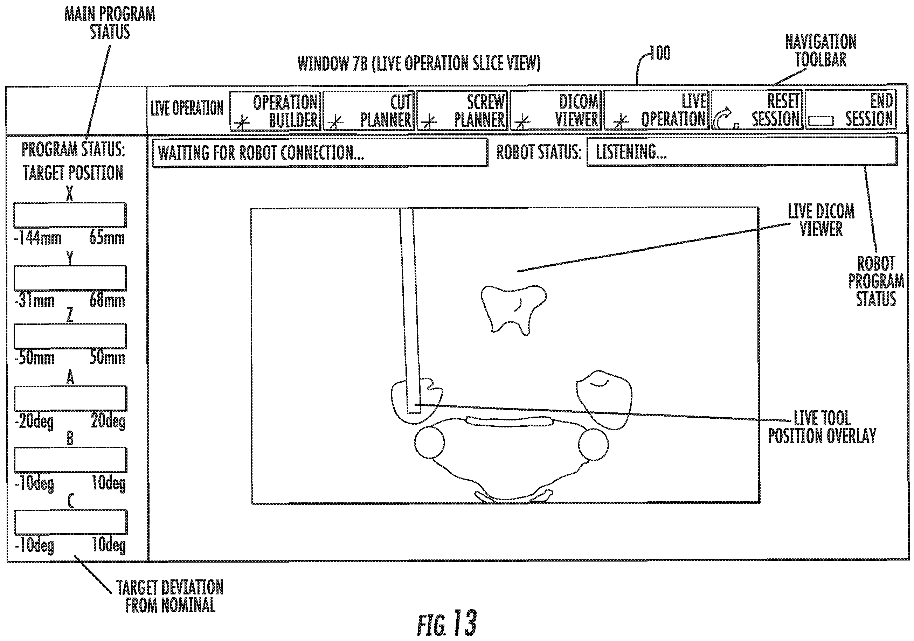

Referring to Figures 10-14, the digital imaging and communications in medicine "DICOM" viewer screens 94, 96, as well as the operation overview screens 98, 100, are illustrated. In these screens, patient 3D simulated imaging in the DICOM format is loaded into the control system 10 to facilitate angled slice viewing. A 3D simulated image is often simply referred to as a 3D image, even though it is displayed on a two dimensional screen. Tools 92 and surgical tool paths 60 for each selected tool can be overlaid upon the DICOM data to show precisely where the tools will enter and traverse according to the tool paths and tools that have been selected and set up for the operation (Figures 12 and 13). Electromagnetic or optical sensors may be utilized to align the DICOM data with the tool paths for robot operation. In this manner, the operator 68 can see precisely where cuts will be made and the depth to which the tools will travel in the anatomy of the patient. This allows preliminary run through of the surgery to allow for modification if desired. In at least one embodiment, the control system 10 provides automatic segmentation and calibration of the DICOM data. In this embodiment, the control system 10 will automatically correlate previously taken DICOM data to the current patient on the table to facilitate live surgical tool path generation and tracking. Either anchor positions or ultrasound sweeping, or a combination of both, will allow the robot 12 to build up precision, relating the DICOM data to the real time patient data until a sufficient match is achieved to begin the operation. This construction also allows minimally invasive surgery "pathing"; whereby the control system includes path generation and planning software that provides for a minimally invasive mode. This mode will optimize robot tool trajectories so that the smallest possible incision (ideally just a single puncture) can be used. Both surface and/or internal tissue damage can be minimized or preferentially protected as per the operator's discretion.

## Claims

1. A robotic surgical system (10) comprising:
a multi-axis surgical robot (12) with a plurality of movable components and operable to hold and operate at least one surgical tool (92);
a computer system (11) having a processor (84), a display device (20, 22) and an input device, said computer system (11) being operably interfaced with said robot (12) for controlling operation of said at least one surgical tool (92) and movement of robot components to effect movement of said at least one surgical tool (92) , said computer system (11) being configured to receive one or more image files of one or more pictures of a surgical site and display at least one said picture upon command, said display (20, 22) being operable to receive information from a user about a proposed surgical tool path (60) overlaid on at least one said picture for at least one said surgical tool (92), said proposed path instruction being processed by a processor (84) and used to direct movement of said at least one surgical tool (92) to perform a surgical procedure at said surgical site,
wherein said display (20, 22) includes a touch screen (18) operable to function as said input device, and the information is input by a user touching said touch screen (18), and said computer system (11) being operable to display the input information for viewing by said user,
wherein said processor (84) determines said surgical tool path (60) and provides surgical tool path data to said robot (12) to effect movement of said tool (92) along said movement path,
wherein said processor (84) being configured to determine movement of said robot components to effect movement of said surgical tool (92) along said surgical tool path (60),
wherein said processor (84) being configured to analyse said proposed surgical tool path (60) and effect changes to said surgical tool path (60),
**characterised by**
said computer system (11) being configured to receive instructions on surgical tool (92) selection from said user, the surgical tool path (60) being modified by the processor (84) to generate a revised surgical tool path for the selected surgical tool (92).

2. The system of Claim 1 wherein said computer system (11) being configured to determine the location of said surgical tool (92) relative to said surgical site prior to surgical operation using said surgical tool (92).

3. The system of Claim 1 wherein said computer system (11) being configured to display an operation builder screen having a plurality of selectable operation procedures for selection to at least partially program operation of said robot (12) and said surgical tool (92).

4. The system of Claim 1 wherein said computer system (11) being configured to display at least one simulated image of said surgical site and show a currently predicted surgical tool path (60) and surgical tool (92) location on the image and allow said user to adjust a current predicted path to a new predicted path, said image including a simulated 3D image.

5. The system of Claim 4 wherein said computer system (11) displaying at least one image of said surgical site and showing a currently proposed surgical path (60) and surgical tool (92) location on the image and allowing at least one of said user and said computer system (11) to adjust said proposed surgical path (60) to a revised surgical path.

6. The system of Claim 5 wherein the at least one image including a simulated 3D image.

7. The system of Claim 5 wherein said surgical procedure being an orthopedic surgery procedure.

8. The system of Claim 1 wherein said surgical site includes at least one electromagnetic sensor in electrical communication with said computer system (11) for aligning said surgical site picture with a surgical site for control positioning of said multi-axis surgical robot (12).

9. The system of Claim 2 wherein said information displayed as the proposed surgical path (60) on the touch screen (18) for viewing by said user.

## Patentansprüche

1. Chirurgisches Robotersystem (10), umfassend:
- einen mehrachsigen chirurgischen Roboter (12) mit einer Mehrzahl beweglicher Komponenten, der bedienbar ist, um zumindest ein chirurgisches Instrument (92) zu halten und zu bedienen;
- ein Computersystem (11) mit einem Prozessor (84), einer Anzeigevorrichtung (20, 22) und einer Eingabevorrichtung, wobei das Computersystem (11) betriebsmäßig mit dem Roboter (12) verbunden ist, um den Betrieb von zumindest einem chirurgischen Instrument (92) und die Bewegung der Roboterkomponenten zu steuern, um die Bewegung von zumindest einem chirurgischen Instrument (92) zu bewirken, wobei das Computersystem (11) eingerichtet ist, um eine oder mehrere Bilddateien eines oder mehrerer Bilder einer Operationsstelle zu empfangen und zumindest ein Bild auf Anweisung anzuzeigen, wobei die Anzeige (20, 22) bedienbar ist, um Informationen von einem Benutzer über einen vorgeschlagenen chirurgischen Instrumentenweg (60) zu empfangen, der über zumindest einem Bild für zumindest ein chirurgisches Instrument (92) liegt, wobei die vorgeschlagenen Weganweisung von einem Prozessor (84) verarbeitet und verwendet wird, um die Bewegung des zumindest einen chirurgischen Instrument (92) zu steuern, um einen chirurgischen Eingriff an der Operationsstelle durchzuführen,
- wobei die Anzeige (20, 22) einen Touchscreen (18) umfasst, der als Eingabevorrichtung fungiert, und die Informationen durch Berühren des Touchscreens (18) durch einen Benutzer eingegeben werden, und wobei das Computersystem (11) bedienbar ist, um die eingegebenen Informationen zur Ansicht für den Benutzer anzuzeigen,
- wobei der Prozessor (84) den chirurgischen Instrumentenweg (60) bestimmt und Daten des chirurgischen Instrumentenwegs an den Roboter (12) vorsieht, um die Bewegung des Instruments (92) entlang des Bewegungswegs zu bewirken,
- wobei der Prozessor (84) eingerichtet ist, um die Bewegung der Roboterkomponenten zu bestimmen, um die Bewegung des chirurgischen Instruments (92) entlang des chirurgischen Instrumentenwegs (60) zu bewirken,
- wobei der Prozessor (84) eingerichtet ist, um den vorgeschlagenen chirurgischen Instrumentenweg (60) zu analysieren und Änderungen am chirurgischen Instrumentenweg (60) vorzunehmen,
- **dadurch gekennzeichnet, dass** das Computersystem (11) eingerichtet ist, um Anweisungen zur Auswahl des chirurgischen Instruments (92) vom Benutzer zu empfangen, wobei der chirurgische Instrumentenweg (60) durch den Prozessor (84) modifiziert wird, um einen überarbeiteten chirurgischen Instrumentenweg für das ausgewählte chirurgische Instrument (92) zu erzeugen.

2. System nach Anspruch 1, wobei das Computersystem (11) eingerichtet ist, um die Position des chirurgischen Instruments (92) relativ zur Operationsstelle vor dem chirurgischen Eingriff unter Verwendung des chirurgischen Instruments (92) zu bestimmen.

3. System nach Anspruch 1, wobei das Computersystem (11) eingerichtet ist, um einen Operationsgenerator-Bildschirm mit einer Mehrzahl von auswählbaren Operationseingriffen anzuzeigen, um den Betrieb des Roboters (12) und des chirurgischen Instruments (92) zumindest teilweise zu programmieren.

4. System nach Anspruch 1, wobei das Computersystem (11) eingerichtet ist, um zumindest ein simuliertes Bild der Operationsstelle anzuzeigen und einen aktuell vorhergesagten chirurgischen Instrumentenweg (60) und eine Position des chirurgischen Instruments (92) auf dem Bild darzustellen und dem Benutzer zu ermöglichen, um einen aktuell vorhergesagten Weg auf einen neuen vorhergesagten Weg einzustellen, wobei das Bild ein simuliertes 3D-Bild umfasst.

5. System nach Anspruch 4, wobei das Computersystem (11) zumindest ein Bild der Operationsstelle anzeigt und einen aktuell vorgeschlagenen Operationsweg (60) und die Position des chirurgischen Instruments (92) auf dem Bild darstellt und zumindest dem Benutzer oder dem Computersystem (11) ermöglicht, den vorgeschlagenen Operationsweg (60) an einen überarbeiteten Operationsweg anzupassen.

6. System nach Anspruch 5, wobei zumindest ein Bild ein simuliertes 3D-Bild umfasst.

7. System nach Anspruch 5, wobei der chirurgische Eingriff ein orthopädischer chirurgischer Eingriff ist.

8. System nach Anspruch 1, wobei die Operationsstelle zumindest einen elektromagnetischen Sensor umfasst, der in elektrischer Verbindung mit dem Computersystem (11) steht, um das Bild der Operationsstelle mit einer Operationsstelle zur Steuerung einer Positionierung des mehrachsigen chirurgischen Roboters (12) auszurichten.

9. System nach Anspruch 2, wobei die Informationen als vorgeschlagener Operationsweg (60) auf dem Touchscreen (18) zur Ansicht durch den Benutzer angezeigt werden.

## Revendications

1. Système chirurgical robotisé (10) comprenant :
un robot chirurgical multiaxial (12) comportant une pluralité de composants mobiles et pouvant maintenir et actionner au moins un outil chirurgical (92) ;
un système informatique (11) comportant un processeur (84), un dispositif d'affichage (20, 22) et un dispositif d'entrée, ledit système informatique (11) étant interfacé de manière opérationnelle avec ledit robot (12) pour commander le fonctionnement dudit au moins un outil chirurgical (92) et le déplacement des composants de robot afin de provoquer le déplacement dudit au moins un outil chirurgical (92), ledit système informatique (11) étant configuré pour recevoir un ou plusieurs fichiers image correspondant à une ou plusieurs images d'un site chirurgical et pour afficher au moins une desdites images sur commande, ledit dispositif d'affichage (20, 22) pouvant être utilisé pour recevoir des informations d'un utilisateur concernant une trajectoire proposée (60) d'outil chirurgical, superposée sur au moins l'une desdites images pour au moins un dit outil chirurgical (92), ladite instruction de trajectoire proposée étant traitée par un processeur (84) et utilisée pour diriger le déplacement dudit au moins un outil chirurgical (92) afin de réaliser une intervention chirurgicale au niveau dudit site chirurgical,
dans lequel ledit dispositif d'affichage (20, 22) comprend un écran tactile (18) pouvant fonctionner en tant que ledit dispositif d'entrée, et les informations sont entrées par un utilisateur qui touche ledit écran tactile (18), et ledit système informatique (11) étant conçu pour afficher les informations entrées afin que ledit utilisateur puisse les visualiser,
dans lequel ledit processeur (84) détermine ladite trajectoire (60) d'outil chirurgical et transmet les données de trajectoire d'outil chirurgical audit robot (12) afin de provoquer le déplacement dudit outil (92) le long de ladite trajectoire de déplacement,
dans lequel ledit processeur (84) est configuré pour déterminer le déplacement desdits composants de robot afin de provoquer le déplacement dudit outil chirurgical (92) le long de ladite trajectoire (60) d'outil chirurgical,
dans lequel ledit processeur (84) est configuré pour analyser ladite trajectoire proposée (60) d'outil chirurgical et pour apporter des modifications à ladite trajectoire (60) d'outil chirurgical,
**caractérisé par**
ledit système informatique (11) étant configuré pour recevoir de la part dudit utilisateur des instructions relatives au choix d'un outil chirurgical (92), la trajectoire (60) d'outil chirurgical étant modifiée par le processeur (84) afin de générer une trajectoire révisée d'outil chirurgical pour l'outil chirurgical choisi (92).

2. Système selon la revendication 1, dans lequel ledit système informatique (11) est configuré pour déterminer la position dudit outil chirurgical (92) par rapport audit site chirurgical avant l'intervention chirurgicale utilisant ledit outil chirurgical (92).

3. Système selon la revendication 1, dans lequel ledit système informatique (11) est configuré pour afficher un écran de création d'intervention comportant une pluralité de procédures d'intervention sélectionnables, destinées à programmer au moins partiellement le fonctionnement dudit robot (12) et dudit outil chirurgical (92).

4. Système selon la revendication 1, dans lequel ledit système informatique (11) est configuré pour afficher au moins une image simulée dudit site chirurgical et pour montrer sur l'image la trajectoire actuellement prévue (60) d'outil chirurgical et la position de l'outil chirurgical (92), et permettre audit utilisateur de modifier la trajectoire actuellement prévue pour obtenir une nouvelle trajectoire prévue, ladite image comprenant une image 3D simulée.

5. Système selon la revendication 4, dans lequel ledit système informatique (11) affiche au moins une image dudit site chirurgical et indique sur l'image la trajectoire chirurgicale actuellement proposée (60) ainsi que la position de l'outil chirurgical (92), et permet à au moins l'un parmi ledit utilisateur et ledit système informatique (11) d'ajuster ladite trajectoire chirurgicale proposée (60) pour obtenir une trajectoire chirurgicale révisée.

6. Système selon la revendication 5, dans lequel ladite au moins une image comprend une image 3D simulée.

7. Système selon la revendication 5, dans lequel ladite intervention chirurgicale est une intervention de chirurgie orthopédique.

8. Système selon la revendication 1, dans lequel ledit site chirurgical comprend au moins un capteur électromagnétique en communication électrique avec ledit système informatique (11) afin d'aligner ladite image de site chirurgical avec un site chirurgical, en vue du positionnement contrôlé dudit robot chirurgical multiaxial (12).

9. Système selon la revendication 2, dans lequel lesdites informations sont affichées sous la forme d'une trajectoire chirurgicale proposée (60) sur l'écran tactile (18) afin que ledit utilisateur puisse les visualiser.
